(19) **Europäisches Patentamt**

**European Patent Office**

**Office européen des brevets**

(11) **EP 1 363 123 A2**

(12) **EUROPEAN PATENT APPLICATION**

(43) Date of publication:
**19.11.2003 Bulletin 2003/47**

(51) Int Cl.7: **G01N 33/28**

(21) Application number: **03076292.6**

(22) Date of filing: **29.04.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **17.05.2002 US 150586**

(71) Applicant: **Delphi Technologies, Inc.
Troy, MI 48007 (US)**

(72) Inventors:
• **Budeiri, Fawaz N.
El Paso, TX 79932 (US)**
• **Buelna, Carlos A.
Cd, Juarez, Chihuahua 32540 (MX)**
• **Flores-Mena, Luis A.
Juarez, Chihuahua 32617 (MX)**

(74) Representative: **Denton, Michael John
Delphi European Headquarters,
64 avenue de la Plaine de France,
Paris Nord II,
BP 60059,
Tremblay-en-France
95972 Roissy Charles de Gaulle Cédex (FR)**

(54) **Method for predicting engine oil contamination**

(57)　A method for determining engine oil contamination includes determining an engine run time and an oil temperature. Based on the oil temperature, a cold event counter is increased by one or a hot event duration value is set equal to the run time of the engine. The cold event counter and the hot event duration value is then used to predict when the engine oil has been contaminated by water and fuel.

Fig.2.

EP 1 363 123 A2

## Description

TECHNICAL FIELD

The present invention relates generally to engine oil condition sensors.

BACKGROUND OF THE INVENTION

Many modern vehicles are equipped with oil condition sensors that are able to detect the contamination of engine oil caused by certain driving conditions. For example, in a short driving schedule where the engine oil temperature does not rise above sixty to seventy degrees Celsius (60 C - 70 C) water and fuel can accumulate in the engine oil and not be evaporated. Furthermore, for vehicles operating in extreme cold conditions, it can take a relatively long time for the engine oil to reach a temperature sufficient to evaporate water and fuel in the engine lubricating system.

It happens that certain current state-of-the art oil condition sensors can include an algorithm that is used to detect a sudden and consistent rise in oil conductivity and trigger a contamination warning when the conductivity value eclipses a certain threshold. Unfortunately, as recognized by the present invention, there are cases, based on the location of the sensor, e.g., in an engine oil pan, in which the sensor fails to send a signal indicative of engine oil contamination.

Accordingly, the present invention recognizes that there is a need for a reliable method for determining engine oil contamination.

SUMMARY OF THE INVENTION

A method for determining contamination of engine oil includes determining run time of an engine and an oil temperature. Based on the oil temperature, a cold event counter is increased by one. Moreover, based on the oil temperature, a hot event duration value is set equal to the run time of the engine. In turn, based on the cold event counter value and the hot event duration value, a contamination signal is sent to a warning device.

In a preferred embodiment, an ignition event counter value and a cold event ratio are determined. The ignition event counter value represents the number of times that the ignition has been turned on and the engine is running. The cold event ratio is determined by CER = (CE/IE) * 100, where CER is the cold event ratio, CE is the cold event counter value, and IE is the ignition event counter value. Preferably, a forgettable factor is determined based on the hot event duration value. The forgettable factor can be determined by FF = (-0.142 * HED) + 1.0715, where FF is the forgettable factor and HED is the hot event duration value.

Preferably, a temperature weight factor is determined based on the oil temperature. Specifically, the temperature weight factor can be determined by TWF = (-0.0056 * $T_{oil}$) + 0.88, where TWF is the temperature weight factor and $T_{oil}$ is the oil temperature. In a preferred embodiment, the temperature weight factor is determined "n" times and the mean of the "n" temperature weight factors is determined.

Also, an actual state of contamination value is determined by the equation ASC = (CER * $TWF_m$) * FF, where ASC is the actual state of contamination, CER is the cold event ratio, $TWF_m$ is the mean of the "n" temperature weight factors, and FF is the forgettable factor. A contamination trigger threshold is determined by the equation CTT = (-1.25 * ASC) + 175, where CTT is the contamination trigger threshold and ASC is the actual state of contamination value. Preferably, the actual state of contamination value is compared to the contamination trigger threshold. Based on this comparison, a contamination signal is sent to warning device. In a preferred embodiment, the contamination signal is reset when the engine oil is changed.

In another aspect of the present invention, an oil sensor apparatus includes an oil condition sensor and a control module. The control module outputs a contamination signal based on a signal from the sensor or based on a default contamination determination based at least in part on cold engine starts.

In yet another aspect of the present invention, an oil sensor apparatus includes an oil condition sensor and a control module connected to the oil condition sensor. In this aspect of the present invention, the control module includes logic means for determining run time of an engine, logic means for determining an oil temperature, logic means for increasing a cold event counter by one based on the oil temperature, logic means for setting a hot event duration value equal to the run time of the engine based on the oil temperature, and logic means for outputting a contamination signal at least partially based on the cold event counter value and the hot event duration value.

The present invention will now be described, by way of example, with reference to the accompanying drawings, in which:

BRIEF DESCRIPTION OF THE DRAWINGS

Figure 1 is a block diagram of a vehicle control system;
Figure 2 is a flow chart of a method for determining engine oil contamination;
Figure 3 is a flow chart of a method for determining engine oil contamination;
Figure 4 is a graph of a forgettable factor versus an elapsed hot event duration;
Figure 5 is a graph of a temperature weight factor versus oil temperature; and
Figure 6 is a graph of a contamination trigger threshold versus an oil contamination percentage.

DESCRIPTION OF AN EMBODIMENT OF THE INVENTION

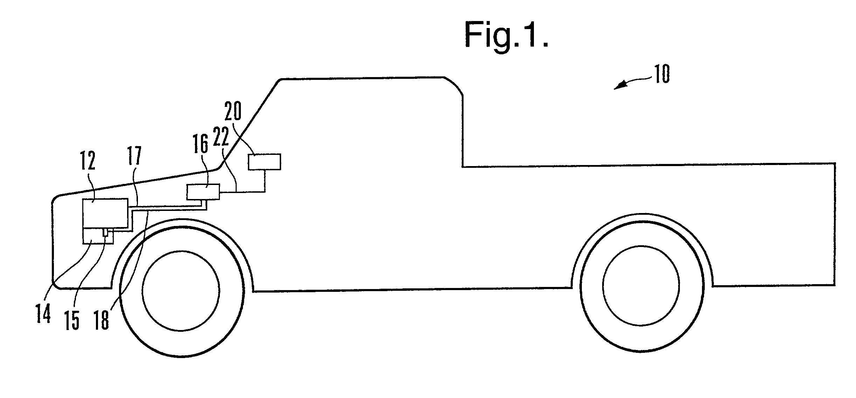

Referring initially to Figure 1, a vehicle is shown and generally designated 10. Figure 1 shows that the vehicle 10 includes an engine 12 that is in fluid communication with an oil reservoir 14, e.g., an oil pan. An oil condition sensor 15 is placed within the oil reservoir 14. As shown, a control module 16, e.g., a vehicle control module (VCM) or body control module (BCM), is electrically connected to the engine 12 via electrical line 17. The control module 16 is also electrically connected to the sensor 15 via electrical line 18. A warning device 20, e.g., a visual warning device or audible warning device, is electrically connected to the control module 16 via electrical line 22. It is to be understood that the control module 16 can be a microprocessor that includes a series of computer-executable instructions, as described below, which will allow the control module 16 to predict when engine oil in the oil reservoir 14 is contaminated with water and fuel based on the operation of the engine 12. These instructions may reside, for example, in the control module 16, which, when programmed with the present logic, establishes a computer program product.

Alternatively, the instructions may be contained on a data storage device with a computer readable medium, such as a computer diskette having a data storage medium holding computer program code elements. Or, the instructions may be stored on, magnetic tape, conventional hard disk drive, electronic read-only memory, optical storage device, or other appropriate data storage device. In an illustrative embodiment of the invention, the computer-executable instructions may be lines of compiled C++ compatible code. As yet another equivalent alternative, the logic can be embedded in an application specific integrated circuit (ASIC) chip or other electronic circuitry.

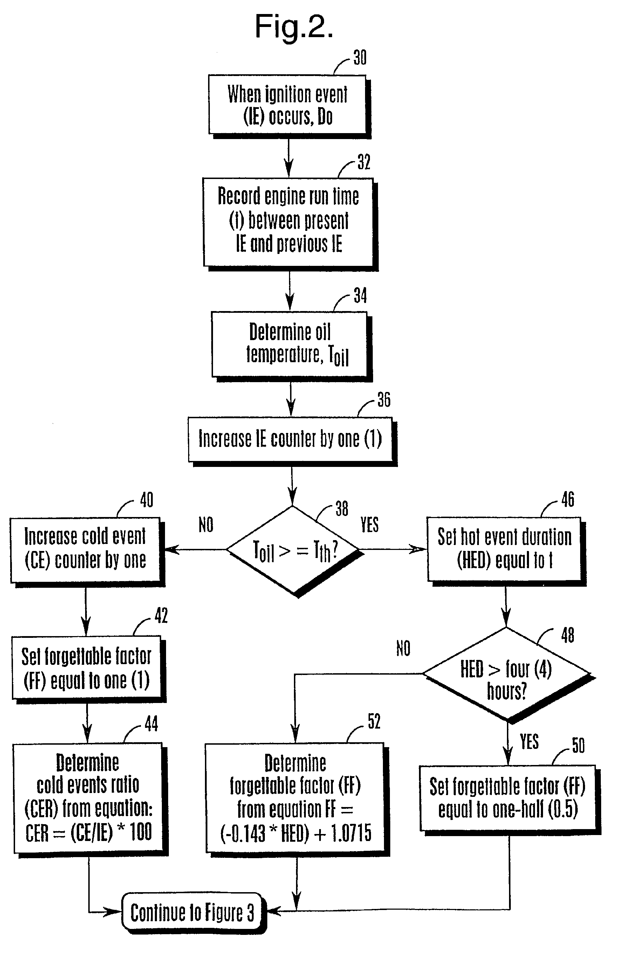

Referring now to Figure 2, the method for determining engine oil contamination is shown and commences at block 30 with a do loop wherein when an ignition event (IE) occurs, the following steps are performed. It is to be understood that an IE is an ignition on to off transition, i.e., the engine is running and then, it is turned off. At block 32, the engine run time, t, between the present IE and the previous IE is recorded. Moving to block 34, the oil temperature, $T_{oil}$, immediately following the present IE is determined. Next, at block 36 an IE counter is increased by one (1).

Proceeding to decision diamond 38, it is determined whether $T_{oil}$ is greater than or equal to a predetermined temperature threshold, $T_{th}$, at which water and/or fuel begin to evaporate from the engine oil. If $T_{oil}$ is less than $T_{th}$, a cold event (CE) has occurred and the logic continues to block 40 where a CE counter is increased by one (1). Thereafter, at block 42, a forgettable factor (FF), used below, is initialized equal to one (1). Moving to block 44 a cold events ratio (CER) is determined from the equation:

$$CER = (CE/IE) * 100,$$

where

CER = cold events ratio,
CE = number of cold events, and
IE = number of ignition events.

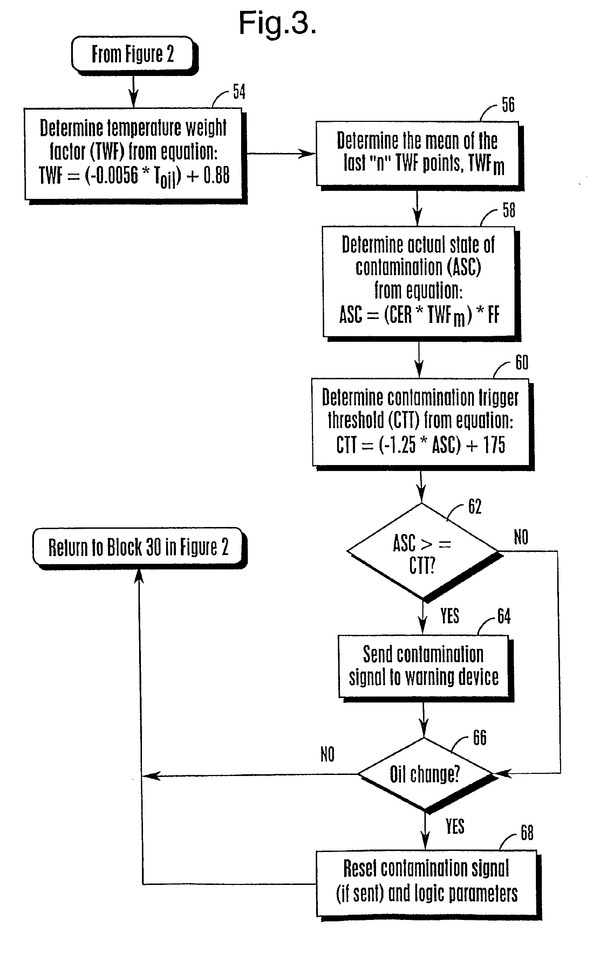

The logic then proceeds to block 54 of Figure 3, described below.

Returning to decision diamond 38, if $T_{oil}$ is greater than or equal to $T_{th}$, a hot event (HE) has occurred and the logic moves to block 46 where a hot event duration (HED) value is set equal to t. Continuing to decision diamond 48, it is determined whether HED is greater than four hours (4 hrs). If so, the logic proceeds to block 50 and FF is set equal to one-half (0.5). The logic then moves to block 54 of Figure 3, described below.

On the other hand, at decision diamond 48, if HED is less than four hours (4 hrs), the logic moves to block 52. At block 52, FF is determined from the equation:

$$FF = (-0.142 * HED) + 1.0715$$

where,

FF = forgettable factor, and
HED = hot event duration (hrs).

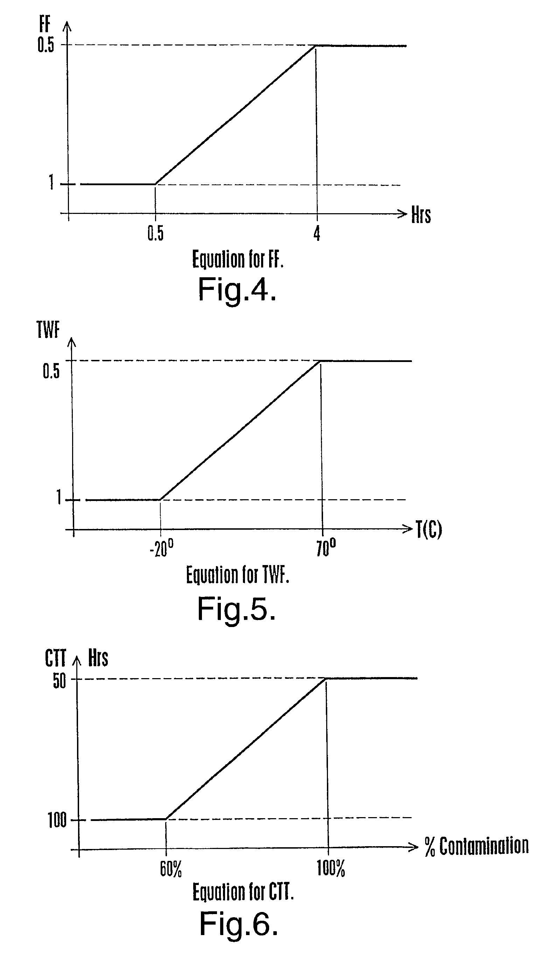

It is to be understood that the above equation for FF is shown graphically in Figure 4 and in this exemplary, non-limiting embodiment, the equation for FF is simply a straight-line relationship between FF and HED. Following the determination of FF at block 52, the logic moves to block 54, described below in conjunction with Figure 3.

Referring now to Figure 3, the logic continues at block 54 where a temperature weight factor (TWF) is determined from the equation:

$$TWF = (-0.0056 * T_{oil}) + 0.88$$

where,

TWF = temperature weight factor, and
$T_{oil}$ = temperature of the engine oil following the IE ( C).

It is to be understood that the above equation for TWF is shown graphically in Figure 5 and in this exemplary, non-limiting embodiment, the equation for TWF is simply a straight-line relationship between TWF and $T_{oil}$.

Moving to block 56, the mean of the last "n" TWF, $TWF_m$, values is determined. Next, at block 58, an actual state of contamination is determined from the equation:

$$ASC = (CER * TWF_m) * FF$$

where,

ASC = actual state of contamination,
CER = cold event ratio,
$TWF_m$ = mean temperature weight factor, and
FF = forgettable factor.

Proceeding to block 60, a contamination trigger threshold (CTT) is determined from the equation:

$$CTT = (-1.25 * ASC) + 175$$

where,

CTT = contamination trigger threshold, and
ASC = actual state of contamination.

It is to be understood that the above equation for CTT is shown graphically in Figure 6 and in this exemplary, non-limiting embodiment, the equation for CTT is simply a straight-line relationship between CTT and ASC.

Next, at decision diamond 62 it is determined whether ASC is greater than or equal to CTT. If so, the logic moves to block 64 and a contamination signal is sent from the control module 16 to the warning device 20. The logic then moves to decision diamond 66, described below. At decision diamond 62 if ASC is less than CTT, the logic moves to decision diamond 66 where it is determined whether the engine oil has been changed. If not, the logic returns to block 30 in Figure 2. On the other hand, if the oil has been changed, the logic moves to block 68 where the contamination signal is reset and all relevant logic parameters are reset. The logic then returns to block 30 in Figure 2.

With the logic steps described above, the method of the present invention can reliably determine when the engine oil has been contaminated by water and fuel based on the operation and run time of the engine 12.

While the particular METHOD FOR DETERMINING ENGINE OIL CONTAMINATION as herein shown and described in detail is fully capable of attaining the above-described objects of the invention, it is to be understood that it is the presently preferred embodiment of the present invention and thus, is representative of the subject matter which is broadly contemplated by the present invention, that the scope of the present invention fully encompasses other embodiments which may become obvious to those skilled in the art, and that the scope of the present invention is accordingly to be limited by nothing other than the appended claims, in which reference to an element in the singular is not intended to mean "one and only one" unless explicitly so stated, but rather "one or more." All structural and functional equivalents to the elements of the above-described preferred embodiment that are known or later come to be known to those of ordinary skill in the art are expressly incorporated herein by reference and are intended to be encompassed by the present claims. Moreover, it is not necessary for a device or method to address each and every problem sought to be solved by the present invention, for it is to be encompassed by the present claims. Furthermore, no element, component, or method step in the present disclosure is intended to be dedicated to the public regardless of whether the element, component, or method step is explicitly recited in the claims. No claim element herein is to be construed under the provisions of 35 U.S.C. section 112, sixth paragraph, unless the element is expressly recited using the phrase "means for."

**Claims**

1. A method for determining contamination of engine oil comprising the acts of:

   determining run time of an engine (12);
   determining an oil temperature;
   based on the oil temperature, increasing a cold event counter by one;
   based on the oil temperature, setting a hot event duration value equal to the run time of the engine; and
   at least partially based on the cold event counter value and the hot event duration value, sending a contamination signal to a warning device (20).

2. The method of Claim 1, further comprising the act of:

   determining an ignition event counter value.

3. The method of Claim 2, further comprising the act of:

   determining a cold event ratio.

4. The method of Claim 3, wherein the cold event ratio is determined by CER = (CE/IE) * 100, where CER is the cold event ratio, CE is the cold event counter value, and IE is the ignition event counter value.

**5.** The method of Claim 4, further comprising the act of:

> based on the hot event duration value determining a forgettable factor.

**6.** The method of Claim 5, wherein the forgettable factor is determined by FF = (-0.142 * HED) + 1.0715, where FF is the forgettable factor and HED is the hot event duration value.

**7.** The method of Claim 6, further comprising the act of:

> based on the oil temperature, determining temperature weight factor.

**8.** The method of Claim 7, wherein the temperature weight factor is determined by TWF = (-0.0056 * $T_{oil}$) + 0.88, where TWF is the temperature weight factor and $T_{oil}$ is the oil temperature.

**9.** The method of Claim 8, further comprising the act of:

> determining the temperature weight factor "n" times.

**10.** The method Claim 9, further comprising the act of:

> determining the mean of the "n" temperature weight factors.

**11.** The method of Claim 10, further comprising the act of:

> determining an actual state of contamination value.

**12.** The method of Claim 11, wherein the actual state of contamination value is determined by the equation ASC = (CER * $TWF_m$) * FF, where ASC is the actual state of contamination, CER is the cold event ratio, $TWF_m$ is the mean of the "n" temperature weight factors, and FF is the forgettable factor.

**13.** The method of Claim 12, further comprising the act of:

> determining a contamination trigger threshold.

**14.** The method of Claim 13, wherein the contamination trigger threshold is determined by the equation CTT = (-1.25 * ASC) + 175, where CTT is the contamination trigger threshold and ASC is the actual state of contamination value.

**15.** The method of Claim 14, further comprising the act of:

> comparing the actual state of contamination value to the contamination trigger threshold.

**16.** The method of Claim 15, further comprising the act of:

> based on the comparison, sending a contamination signal to a warning device.

**17.** The method of Claim 16, further comprising the act of:

> resetting the contamination signal when the engine oil is changed.

**18.** An oil sensor apparatus, comprising:

> at least one oil condition sensor (15); and
> at least one control module (16) outputting a contamination signal based on a signal from the sensor (15) or based on a default contamination determination based at least in part on cold engine starts.

**19.** The oil sensor apparatus of Claim 18, wherein the sensor (15) is installed within an oil reservoir (14).

**20.** The oil sensor apparatus of Claim 19, further comprising:

> a warning device (20) electrically connected to the control module (16).

**21.** An oil sensor apparatus, comprising:

> at least one oil condition sensor (15);
> at least one control module (16) connected to the oil condition sensor (15), the control module (16) having logic means for determining run time of an engine, logic means for determining an oil temperature, logic means for increasing a cold event counter by one based on the oil temperature, logic means for setting a hot event duration value equal to the run time of the engine based on the oil temperature, and logic means for outputting a contamination signal at least partially based on the cold event counter value and the hot event duration value.

**22.** The oil sensor apparatus of Claim 21, wherein the sensor (15) is installed within an oil reservoir (14).

**23.** The oil sensor apparatus of Claim 22 further comprising:

> a warning device (20) electrically connected to

**EP 1 363 123 A2**

the control module (16).

Fig.1.

# Fig.2.

```
                          ┌─────────────────────┐  30
                          │  When ignition event │
                          │  (IE) occurs, Do     │
                          └─────────────────────┘
                                    │
                                    ▼
                          ┌─────────────────────┐  32
                          │  Record engine run   │
                          │  time (t) between    │
                          │  present IE and      │
                          │  previous IE         │
                          └─────────────────────┘
                                    │
                                    ▼
                          ┌─────────────────────┐  34
                          │  Determine oil       │
                          │  temperature, T_oil  │
                          └─────────────────────┘
                                    │
                                    ▼
                          ┌─────────────────────┐  36
                          │ Increase IE counter  │
                          │ by one (1)           │
                          └─────────────────────┘
                                    │
                                    ▼
```

Step 30: When ignition event (IE) occurs, Do

Step 32: Record engine run time (t) between present IE and previous IE

Step 34: Determine oil temperature, $T_{oil}$

Step 36: Increase IE counter by one (1)

Decision 38: $T_{oil} >= T_{th}$?

NO → Step 40: Increase cold event (CE) counter by one

YES → Step 46: Set hot event duration (HED) equal to t

Step 42: Set forgettable factor (FF) equal to one (1)

Step 44: Determine cold events ratio (CER) from equation: $CER = (CE/IE) * 100$

Decision 48: HED > four (4) hours?

NO → Step 52: Determine forgettable factor (FF) from equation $FF = (-0.143 * HED) + 1.0715$

YES → Step 50: Set forgettable factor (FF) equal to one-half (0.5)

Continue to Figure 3

# Fig.3.

From Figure 2

Determine temperature weight
factor (TWF) from equation:
$TWF = (-0.0056 * T_{oil}) + 0.88$

54

Determine the mean of the
last "n" TWF points, $TWF_m$

56

Determine actual state of
contamination (ASC)
from equation:
$ASC = (CER * TWF_m) * FF$

58

Determine contamination trigger
threshold (CTT) from equation:
$CTT = (-1.25 * ASC) + 175$

60

ASC >=
CTT?

62

NO

YES

Send contamination
signal to warning device

64

Return to Block 30 in Figure 2

Oil change?

66

NO

YES

Reset contamination signal
(if sent) and logic parameters

68

Equation for FF.

## Fig.4.

Equation for TWF.

## Fig.5.

Equation for CTT.

## Fig.6.